# FASCICULE DE BREVET EUROPEEN

(11) **EP 2 509 589 B1**
(45) Date de publication et mention de la délivrance du brevet: **06.04.2016**
(21) Numéro de dépôt: 10798281.1
(22) Date de dépôt: 07.12.2010
(51) Int. Cl.: A61K 31/137, A61P 31/16, A61K 31/421, A61K 31/542

(54) **COMPOSITIONS ANTIVIRALES PHARMACEUTIQUES OU VÉTÉRINAIRES COMPRENANT LA MIDODRINE OU LA DESGLYMIDODRINE**
PHARMAZEUTISCHE ODER VETERINÄRMEDZINISCHE ANTIVIRALE ZUSAMMENSETZUNGEN, ENTHALTEND MIDODRINE ODER DESGLYMIDODRINE
PHARMACEUTICAL OR VETERINARY ANTIVIRAL COMPOSITIONS COMPRISING MIDODRINE OR DESGLYMIDODRINE

(30) Priorité: 09.12.2009 FR 0958810; 09.12.2009 US 267997 P
(43) Date de publication de la demande: 17.10.2012
(73) Titulaire: Université Claude Bernard Lyon 1, 69100 Villeurbanne (FR); Centre National de la Recherche Scientifique (CNRS), 75016 Paris (FR); Hospices Civils de Lyon, 69002 Lyon (FR)
(72) Inventeur: ROSA-CALATRAVA, Manuel, F-69003 Lyon (FR); DIAZ, Jean-Jacques, F-69200 Venissieux (FR); TEXTORIS, Julien, F-13004 Marseille (FR); JOSSET, Laurence, F-69006 Lyon (FR)
(74) Mandataire: Regimbeau
(86) Numéro de dépôt international: PCT/EP2010/069023
(87) Numéro de publication internationale: WO 2011/069990

(56) Documents cités:
- WO-A1-2008/099175
- WO-A1-2009/065116
- WO-A2-2007/044752
- HAYDEN FREDERICK: "Developing new antiviral agents for influenza treatment: what does the future hold?", CLINICAL INFECTIOUS DISEASES, THE UNIVERSITY OF CHICAGO PRESS, CHICAGO, IL, US LNKD- DOI:10.1086/591851, vol. 48, no. suppl. 1, 1 janvier 2009 (2009-01-01), pages S3-S13, XP008103335, ISSN: 1058-4838
- MCCLELLAN K J ET AL: "MIDODRINE A REVIEW OF ITS THERAPEUTIC USE IN THE MANAGEMENT OF ORTHOSTATIC HYPOTENSION", DRUGS & AGING, ADIS INTERNATIONAL LTD, NZ, vol. 12, no. 1, 1 janvier 1998 (1998-01-01), pages 75-86, XP009016743, ISSN: 1170-229X
- SCHMIDTKE M ET AL: "Do oxymetazoline-containing nasal sprays exhibit an antiviral activity against influenza A virus?", CHEMOTHERAPIE JOURNAL 200512 DE, vol. 14, no. 6, décembre 2005 (2005-12), pages 207-211, XP009132446, ISSN: 0940-6735
- JOSSET L ET AL: "Gene expression signature-based screening identifies new broadly effective influenza A antivirals", PLOS ONE 2010 PUBLIC LIBRARY OF SCIENCE USA LNKD- DOI:10.1371/JOURNAL.PONE.0013169, vol. 5, no. 10, 4 octobre 2010 (2010-10-04), XP002630133, ISSN: 1932-6203
- SUPURAN C T: "Carbonic anhydrases: Novel therapeutic applications for inhibitors and activators", NATURE REVIEWS DRUG DISCOVERY 200802 GB LNKD- DOI:10.1038/NRD2467, vol. 7, no. 2, février 2008 (2008-02), pages 168-181, XP002542810, ISSN: 1474-1776
- GEBHARDT B M ET AL: "Propranolol suppresses reactivation of herpesvirus", ANTIVIRAL RESEARCH, ELSEVIER BV, NL, vol. 27, 1 janvier 1995 (1995-01-01), pages 255-261, XP002348941, ISSN: 0166-3542, DOI: DOI:10.1016/0166-3542(95)00009-B
- REID J L: "UPDATE ON RILMENIDINE: CLINICAL BENEFITS", AMERICAN JOURNAL OF HYPERTENSION, NEW YORK, NY, US, vol. 14, no. 11 II, 1 janvier 2001 (2001-01-01), pages 322S-324S, XP001121530, DOI: DOI:10.1016/S0895-7061(01)02239-7

## Description

L'invention se rapporte à des compositions pour utilisation pour le traitement des infections virales et en particulier pour le traitement des pathologies liées à l'infection par les virus de la grippe chez l'homme et chez les animaux.

Les responsables de la grippe sont les virus influenza qu'on divise en trois genres ou types: A, B et C. A la surface du virus se trouvent deux glycoprotéines qui jouent un rôle important dans l'infection des cellules de l'organisme infecté : l'hémagglutinine (HA) et la neuraminidase (NA). Il existe différents sous-types de virus influenza A selon la nature des glycoprotéines HA et NA à leur surface: 16 types d'HA et 9 types de NA ont été identifiées chez les virus circulants parmi les oiseaux migrateurs marins. Chez l'homme, les virus circulants depuis plusieurs décénies, sont les sous-types H1N1, H2N2 et H3N2, avec des transmissions inter-espèces occasionnelles (de l'animal à l'homme) des virus aviaires H5N1, H7N7, H5N2 et H9N2. On peut ainsi définir les virus influenza selon le type de protéines qu'il possèdent à leur surface.

Comme le souligne la récente émergence d'un nouveau virus grippal pandémique H1N1 d'origine porcine, aviaire et humaine (virus réassortant porcin, aviaire et humain), les virus influenza de type A représentent une menace sérieuse en santé publique. Les pandémies de grippe sont le résultat de cassures antigéniques qui correspondent à l'apparition de virus dotés de nouvelle glycoprotéines de surface (HA et NA) dans la population humaine. Ces cassures peuvent être dues à la transmission directe chez l'homme de virus aviaires (cas des épidémies de H5N1 aviaires hautement pathogènes depuis 2003 en Asie ou de l'épidémie de grippe H7N7 aux Pays-Bas en 2003). Les cassures antigéniques peuvent également être dues à un réarrangement génétique entre des virus aviaires, porcins et humains, le porc jouant le rôle d'hôte intermédiaire. Cette réadaptation génétique est à l'origine de la pandémie actuelle de H1N1. Par ailleurs, les épidémies de grippe saisonnières sont une cause majeure de morbidité et de mortalité accrue, surtout chez les très jeunes, les vieillards, les immunodéprimés et les porteurs de maladies cardio-pulmonaires.

La vaccination reste la pierre angulaire de la prévention de la grippe. Cependant, lors de l'apparition d'un nouveau virus, un délai est nécessaire à sa mise au point (6 à 9 mois) et l'utilisation de médicaments antiviraux doit être envisagée pour le traitement ou la prévention. Les antiviraux actuels sont les inhibiteurs des canaux M2 (Amantadine) et les inhibiteurs de la neuraminidase (Zanamivir et Oseltamivir). L'utilisation de ces médicaments pourrait être limitée par l'apparition rapide de résistance dont on a déjà pû noter l'apparition pour le virus H1N1 pandémique (dérive génétique par accumulation de mutations). De plus, il n'est pas exclu que les nouveaux virus émergents soient également résistants à ces molécules. Enfin, la plupart de ces molécules ne sont pas administrable par voie générale ce qui pose un problème en cas d'infections graves. Il apparaît donc nécessaire de mettre au point de nouveaux antiviraux, possédant un large spectre d'action et plus facile à administrer.

La demande internationale WO 2007/044752 a pour objet des compositions pour le traitement des affections de la peau. Ces compositions comprennent un antagoniste du récepteur alpha-adrenergique tel que la midodrine. Ce document ne comprend aucune divulgation ni aucun enseignement quant à l'utilisation de produits comprenant la midodrine ou la desglymidodrine pour le traitement d'infections virales.

La demande internationale WO 2009/065116 se rapporte à des méthodes pour traiter le purpura avec des compositions comprenant la midodrine et un autre agent actif pouvant être un antiviral. Ce document ne décrit pas l'utilisation de produits comprenant la midodrine ou la desglymidodrine pour le traitement d'infections virales.

Hayden F. dans clinical infectious diseases 2009:48 (Suppl.1) S3 divulgue les agents antiviraux actuellement en développemment pour le traitement de la grippe.

Une solution pour la mise au point de nouvelles thérapeutiques à large spectre est de viser les facteurs cellulaires indispensables à la réplication virale. Cette stratégie a notamment été développée avec un relatif succès pour lutter contre les rétrovirus. Dans le cadre de la présente invention, plusieurs molécules ont été sélectionnées et elles ont été évaluées dans un test cellulaire d'infection virale. Certaines molécules ont présenté un effet antiviral non seulement sur un ensemble de virus influenza de référence (H1N1, H3N2, H5N2, H7N7 et H5N1), mais également sur le virus H1N1 pandémique (A/California/07/2009) et les souches virale de terrain apparentées.

Les composés sélectionnés avaient été décrits en tant que principe actifs dans le traitement de pathologies très éloignées des infections virales. De façon inattendue, il a maintenant été montré que ces composés ont une activité antivirale et en particulier une activité anti-influenza contre différents sous-types de virus de la grippe.

### RESUME DE l'INVENTION

L'invention a pour objet des compositions pharmaceutiques ou vétérinaires pour utilisation pour la prévention ou le traitement des infections virales comprenant au moins un composé choisi parmi la midodrine, et la desglymidodrine.

Dans un mode de réalisation préféré, les compositions pharmaceutiques ou vétérinaires selon l'invention sont pour utilisation pour la prévention ou le traitement des infections par un virus de la grippe (virus influenza A, B et C).

Préférentiellement, les compositions pharmaceutiques ou vétérinaires selon l'invention comprennent en outre un véhicule pharmaceutique approprié.

Préférentiellement, les compositions pharmaceutiques ou vétérinaires selon l'invention comprennent en outre un autre agent anti-viral ou anti-grippal.

De préférence, l'agent anti-viral ou anti-grippal est choisi parmi l'oseltamivir, le zanamivir, le peramivir, l'amantadine, la rimantadine, la ribavirine et l'arbidol.

L'invention se rapporte aussi à un produit comprenant un composé choisi parmi la midodrine, et la desglymidodrine, ainsi qu'un autre agent antiviral comme produit de combinaison pour une utilisation simultanée, séparée ou étalée dans le temps en thérapie, et en particulier dans la prévention et le traitement des infections virales.

L'invention a aussi pour objet l'utilisation d'au moins un composé choisi parmi la midodrine, et la desglymidodrine pour la fabrication d'un médicament pour la prévention ou le traitement de la grippe.

### DESCRIPTION DE L'INVENTION

La présente invention a pour objet des compositions pharmaceutiques ou vétérinaires pour la prévention et le traitement des infections virales chez l'homme et chez l'animal et plus particulièrement pour le traitement de la grippe. Ces compositions comprennent au moins un composé choisi parmi la midodrine, et la desglymidodrine. Ces composés sont connus pour une utilisation dans d'autres applications thérapeutiques sans rapport avec une activité antivirale chez l'homme ou chez l'animal. Il est maintenant démontré que ces composés ont de façon inattendue une activité antivirale et notamment une activité anti-influenza contre différents sous-types de virus de la grippe A et des grippes B et C.

La midodrine a été décrite pour le traitement de l'hypotension orthostatique. Il s'agit d'une prodrogue donnant après hydrolyse la desglymidodrine qui est le métabolite actif de la midodrine.

Les différents sels pharmaceutiquement acceptables de la midodrine ou de la desglymidodrine peuvent être utilisés dans les compositions de la présente invention, de préférence la midodrine se présente sous forme de chlorhydrate de midodrine représenté ci-dessous : 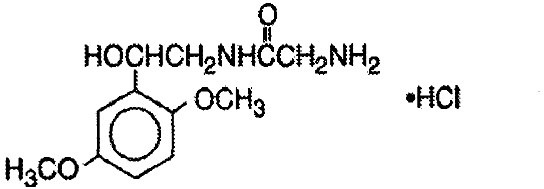 Acetamide, 2-amino-*N*-[2-(2,5-dimethoxyphenyl)-2-hydroxyethyl]-monohydrochloride, (±)-; (2) (±)-2-amino-*N*-(β-hydroxy-2,5-dimethoxyphenethyl)acetamide monohydrochloride

Pour le traitement antiviral d'un adulte, la dose de midodrine administrée est de préférence comprise entre 1mg et 30mg par jour, plus préférentiellement comprise entre 7,5mg et 15 mg par jour.

Les compositions pharmaceutiques ou vétérinaires selon l'invention ont une activité antivirale et conviennent à la prévention ou au traitement de différentes infections virales. En particulier, les compositions pharmaceutiques ou vétérinaires sont utiles pour la prévention ou le traitement des infections par un virus de la grippe (influenza A, B et C).

Avantageusement, les compositions de la présente invention ont un spectre d'action large contre différents types de virus de la grippe (types A, B et C).

Dans un autre mode de réalisation, les compositions de la présente invention sont pour la prévention et le traitement des infections par les virus de type A et de type B.

Dans un autre mode de réalisation, les compositions de la présente invention sont pour la prévention et le traitement des infections par les virus de type A circulants principalement chez l'homme et les animaux.

Dans un autre mode de réalisation, les compositions de la présente invention sont pour la prévention et le traitement des infections par les virus de type B circulants chez l'homme.

Dans un autre mode de réalisation, les compositions de la présente invention sont pour la prévention et le traitement des infections par les virus de type C circulants chez l'homme et les porcs.

L'invention se rapporte ainsi à la prévention et au traitement des infections par les virus de la grippe chez l'homme.

L'invention se rapporte aussi à la prévention et au traitement des infections par les virus de la grippe chez l'animal, en particulier, chez les animaux d'élevage comme les porcs, les chevaux et les volailles. Plus particulièrement, l'invention a pour objet à la prévention et au traitement des infections par les virus influenza chez les volailles et plus particulièrement chez les poules, canards, oies, dindes et dindons. L'invention se rapporte aussi à la prévention et au traitement des infections par les virus de la grippe chez d'autres animaux comme par exemple les chats, les chiens et les félins.

La présente invention a aussi pour objet la prévention et le traitement d'autres infections virales.

De préférence, les compositions pharmaceutiques ou vétérinaires selon l'invention comprennent un composé actif dans un véhicule pharmaceutique approprié.

Ces compositions peuvent être formulées pour l'administration aux mammifères, y compris l'homme. La posologie varie selon le traitement et selon l'affection en cause. Ces compositions sont typiquement réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, oculaire, locale ou rectale, le composé actif peut être administré sous formes unitaires d'administration, en mélange avec des supports pharmaceutiques classiques, aux animaux ou aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le composé actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le composé actif avec un diluant et en versant le mélange obtenu dans des gélules molles ou dures.

Une préparation sous forme de sirop ou d'élixir peut contenir le composé actif conjointement avec un édulcorant, un antiseptique, ainsi qu'un agent donnant du goût et un colorant approprié.

Les poudres ou les granules dispersibles dans l'eau peuvent contenir le composé actif en mélange avec des agents de dispersion ou des agents mouillants, ou des agents de mise en suspension, de même qu'avec des correcteurs du goût ou des édulcorants.

La midodrine, la desglymidodrine, ou leurs mélanges peuvent être employés en thérapie seuls, ou en combinaison avec au moins un autre agent actif. Il peut s'agir d'adjuvants permettant d'améliorer l'activité des composés, ou encore d'autres actifs connus pour leur emploi dans le traitement des infections virales. De tels agents actifs sont bien connus de l'homme du métier, disponibles dans le commerce ou encore décrits dans des ouvrages de référence comme Le Dictionnaire Vidal. Ces autres agents actifs peuvent en particulier être choisis parmi les actifs appropriés pour le traitement des infections virales et plus particulièrement pour le traitement des infections par les virus influenza.

De préférence, l'agent anti-viral ou anti-grippal est choisi parmi l'osetamivir, le zanamivir, le peramivir, l'amantadine, la rimantadine, la ribavirine et l'arbidol.

L'invention se rapporte aussi à un produit comprenant un composé choisi parmi la midodrine, la desglymidodrine, ainsi qu'un autre agent antiviral comme produit de combinaison pour une utilisation simultanée, séparée ou étalée dans le temps en thérapie, et en particulier dans la prévention et le traitement des infections virales.

L'invention a aussi pour objet l'utilisation d'au moins un composé choisi parmi la midodrine, et la desglymidodrine, pour la fabrication d'un médicament pour la prévention ou le traitement de la grippe.

L'invention se rapporte également à des méthodes thérapeutiques pour le traitement des infections virales et plus particulièrement des infections par un virus influenza chez l'homme dans lesquelles on administre une quantité efficace d'un composé choisi parmi la midodrine, et la desglymidodrine, à un patient.

L'invention se rapporte également à des méthodes thérapeutiques pour le traitement des infections virales et plus particulièrement des infections par un virus influenza chez les animaux dans lesquelles on administre une quantité efficace d'un composé choisi parmi la midodrine, et la desglymidodrine, à un animal. Avantageusement, l'animal est un animal d'élevage tel que par exemple, le porc, le cheval ou encore les volailles (chiens, chat..etc).

### FIGURES

Figure 1 : Détermination des zones de cytotoxicité des molécules
Figure 2 : Évaluation de l'activité anti-virale des molécules sur différents virus
Figure 3 : Évaluation de l'activité des molécules sur les virus et leur entrée cellulaire
Figure 4 : Tableau récapitulatif des propriétés antivirales des molécules

### EXEMPLES

### A. Infection des cellules humaines A549 par des virus influenza A humains et aviaires représentatifs

Les cellules A549 (carcinome pulmonaire) sont maintenues en culture dans un milieu DMEM (milieu modifié Dulbecco, BioWhittaker) complémenté avec 10 % (v/v) de sérum de veau foetal, 2 mM de L- glutamine, 100 U/mL de pénicilline et 100 ug/mL de streptomycine, dans un incubateur à 37°C, à une atmosphère saturée en humidité contenant 5 % de CO2. A confluence, les cellules sont détachées du support par traitement à la trypsine-EDTA et réensemencées dans une fiole de culture contenant 15 ml de milieu frais. Trois jours avant l'étape d'infection, 0,75 10E6 cellules sont réparties par flasques 25 cm2 (T25), en milieu à 10 % de sérum de veau foetal de façon à obtenir 70-80% de confluence lors de l'infection (le nombre de cellules est estimé à 3.106 cellules / T25). Les cellules sont infectées par les différents virus influenza A à une moi 0,1 dans du DMEM, 2 mM de L- glutamine, 100 U/mL de pénicilline et 100 ug/mL de streptomycine, et 0,5 ug/mL de trypsine (5 flasques par virus).

Les virus utilisés sont : les souches de virus influenza A humains (H1N1 A/New Caledonia/20/99, H3N2 A/Moscow/10/99) et aviaires (H5N1 A/Turkey/582/2006, H5N2 A/Finch/England/2051/94, H7N1 A/Chicken/Italy/2076/99).

### B. Test de cytotoxicité et évaluation de l'activité anti-virale des molécules

Les molécules potentiellement antivirales ont été testée *ex vivo* sur cellules A549. Toutes les molécules ont été dissoutes dans l'eau sauf la rilmenidine reprise dans du DMSO et le brinzolamide testé avec ses excipients dans le collyre Azopt.

Ces molécules sont testées sur les cellules A549 à 100% de confluence en plaque 96 puits (préparées 3 jours avant à 0,15.105 cellules/puit de manière à n'obtenir 100% de confluence que le jour du test). Les cellules sont incubées pendant 6h avec différentes concentrations de molécules diluées dans 150 uL de milieu d'infection/ puit puis 50 uL/puit de suspension virale sont ajoutés (ou 50 uL de milieu d'infection dans la plaque de cellules non infectées). Un test de cytotoxicité et un test de quantification du virus sont réalisés après 65 h d'incubation à 37°C sous 5% de CO2.

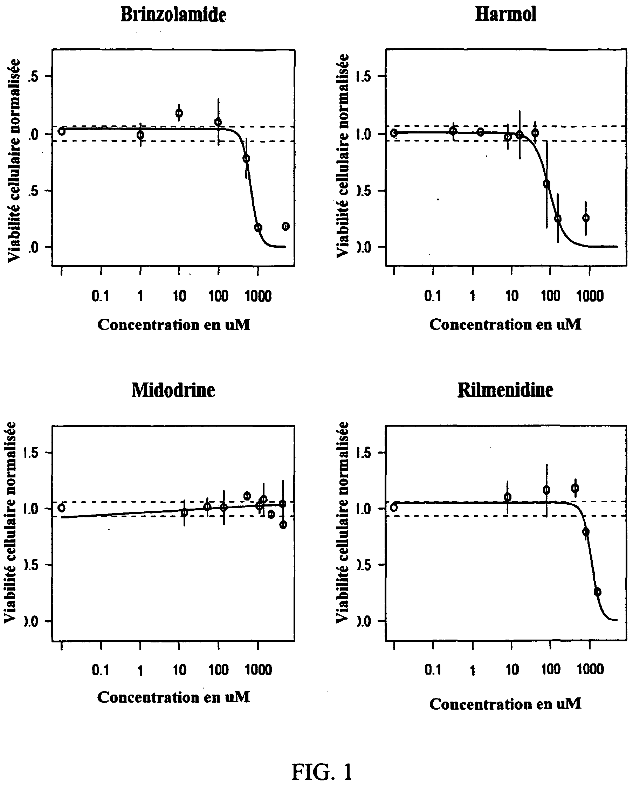

La cytotoxicité des différentes molécules est déterminée à chaque essai dans une plaque de cellules non infectées par un test au rouge neutre. Il s'agit d'un colorant vital des lysosymes. Après 3h d'incubation des cellules en présence de rouge neutre 1,55% et une fixation d'une minute par un mélange Formol/calcium (40%/10%) les cellules sont lysées par de l'acide acétique 2% et l'absorbance est lue à 550 nm. Le rapport de l'absorbance dans chaque puit sur l'absorbance moyenne des puits des cellules témoins (non traitées par les molécules) est calculé et indiqué sur les schémas comme un indice de « Cell Viability ». Les résultats sont représentés molécule par molécule dans la figure 1. Ces résultats sont une compilation de 5 essais indépendants. Ils nous permettent de définir les concentrations de molécules donnant 50% de cytotoxicité (CC50): le brinzolamide à partir de 665,5 mM, l'harmol à 95 mM et la rilmenidine à 1125 mM.

L'effet sur la production virale est testé sur les souches de virus influenza A humains (H1N1 A/New Caledonia/20/99, H3N2 A/Moscow/10/99) et aviaires (H5N1 A/Turkey/582/2006, H5N2 A/Finch/England/2051/94, H7N1 A/Chicken/Italy/2076/99) à 2 multiplicités d'infection (moi) : moi 0,2 et moi 2. Après 65 h d'infection, la quantité de virus produit est estimée par un test d'activité neuraminidase. Pour ce test, 25 uL de suspension virale sont incubés pendant 1h à 37°C avec 75 uL de substrats MUN (à 5 mM) dilué extemporanément au 1/75 dans du tampon réactionnel MES (32,5 mM) CaCl2 (4 mM) pH 5,8. La réaction est stoppée par 150 uL de tampon glycine 50 mM pH 10,4 et révélée par lecture au fluorimètre (λexc 355 nm et λem 460 nm). De la même manière que pour la cytotoxicité, le rapport de l'intensité de fluorescence dans chaque puit sur l'intensité de fluorescence moyenne des puits des cellules témoins (non traitées par les molécules) est calculé et indiqué sur les schémas comme un indice de « Viral Production ».

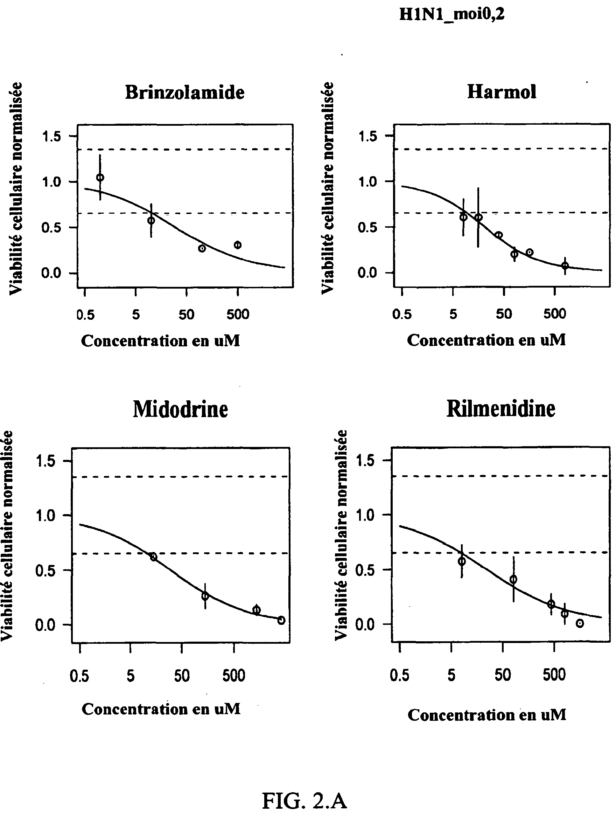

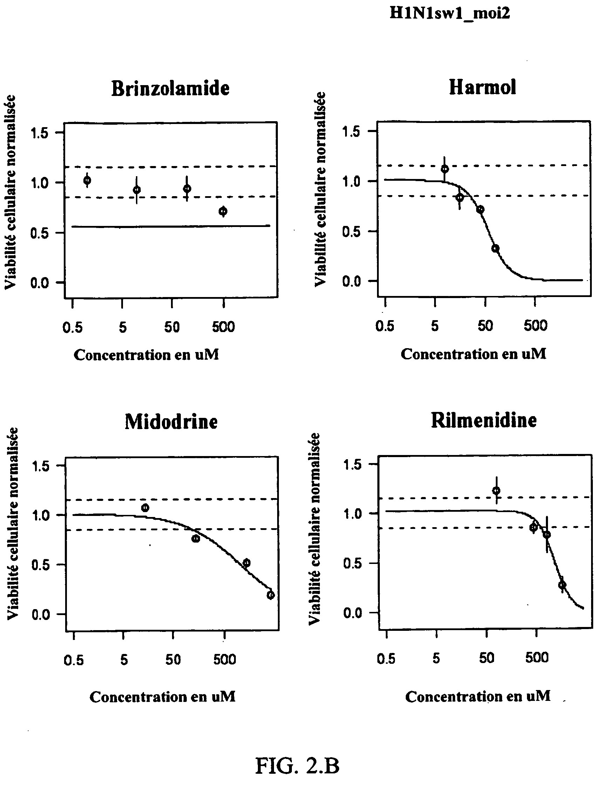

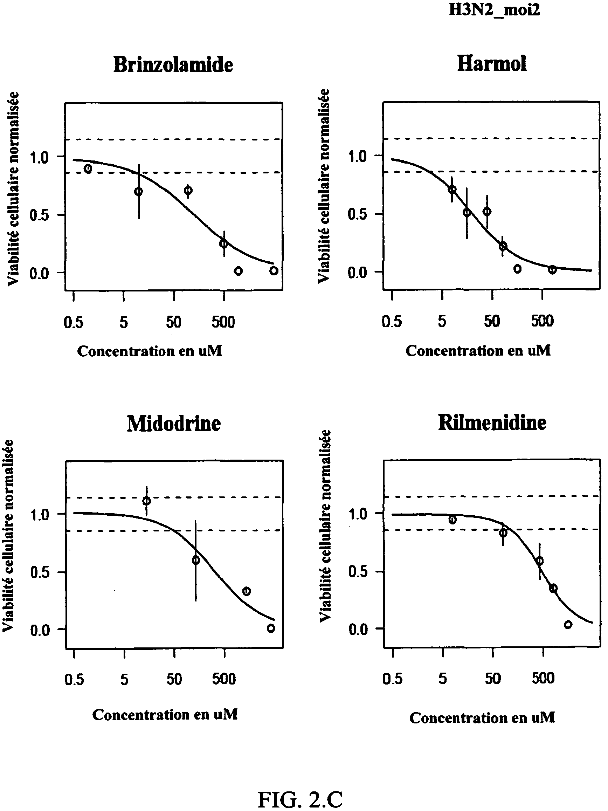

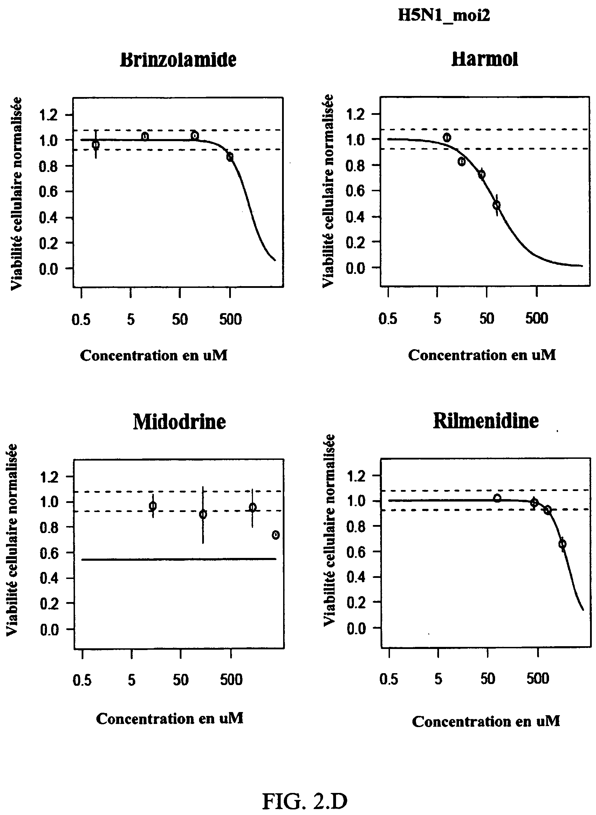

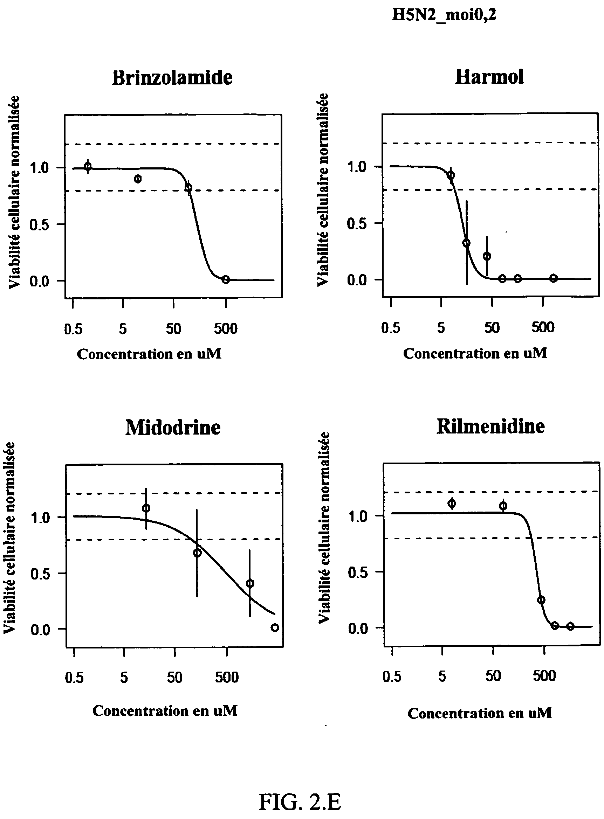

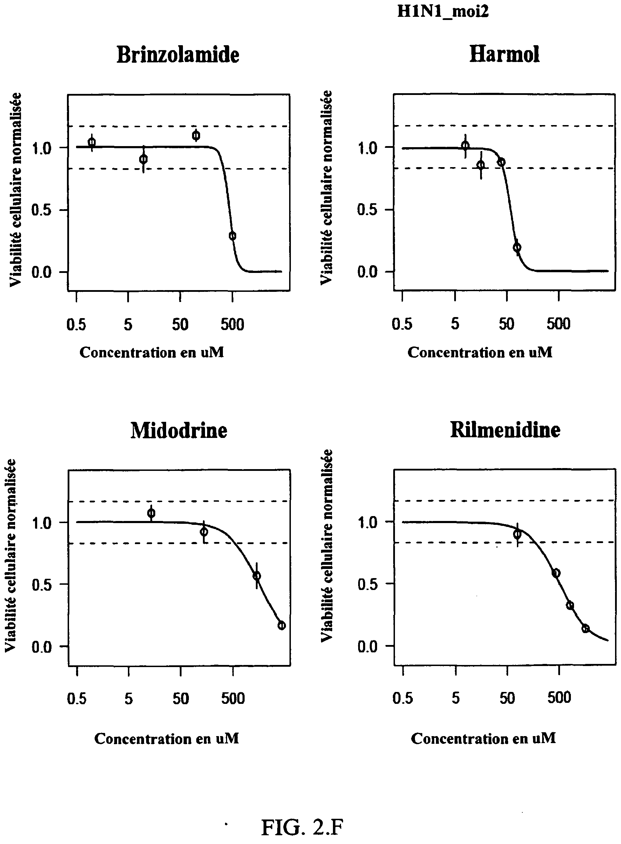

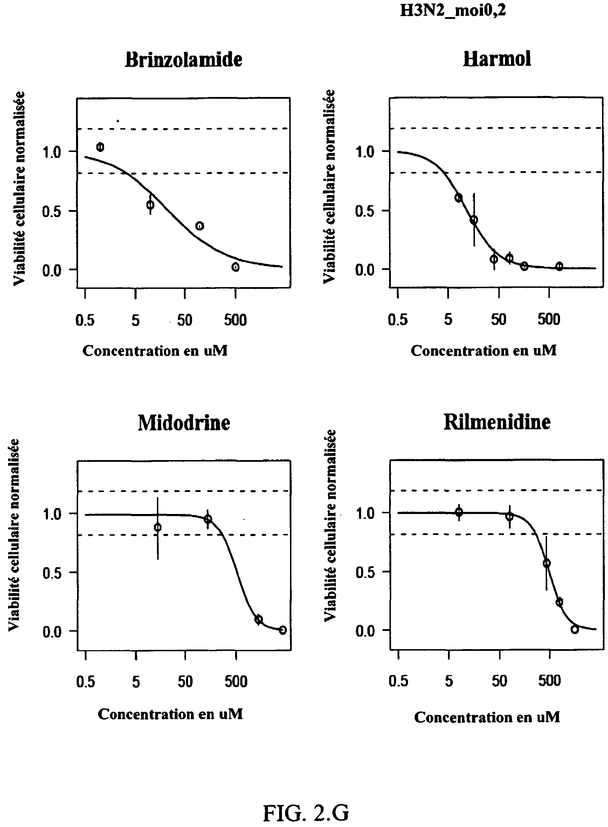

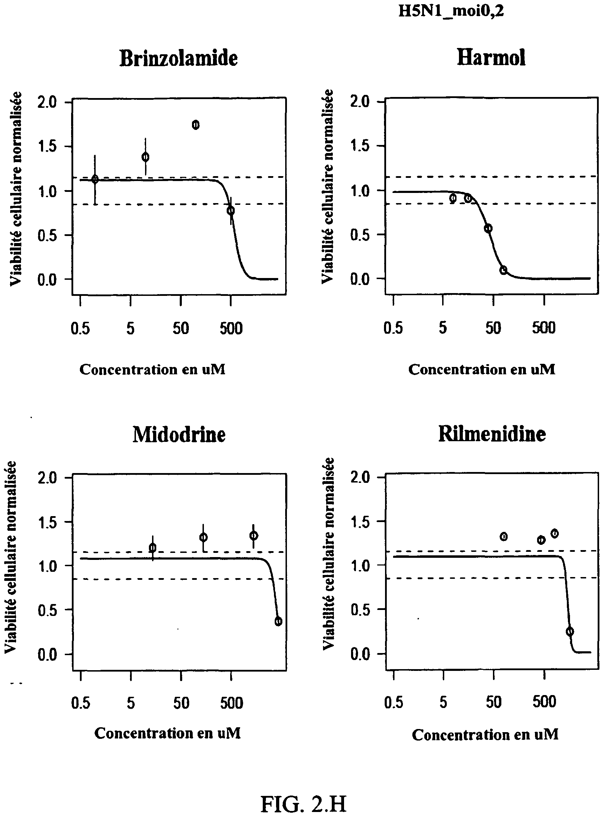

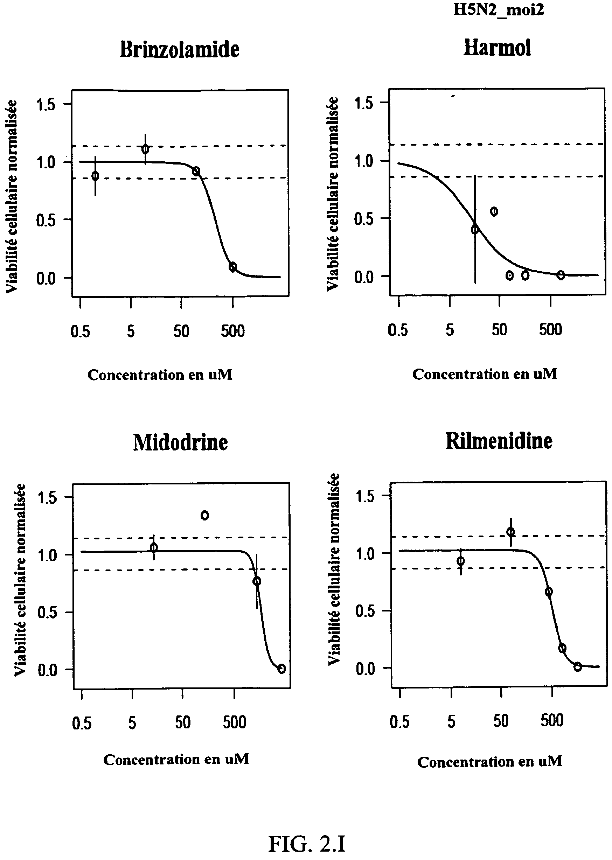

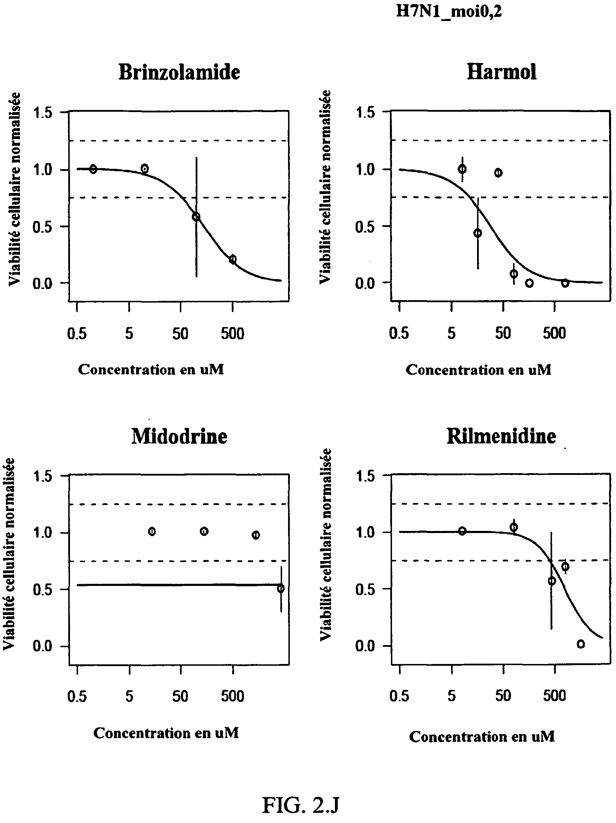

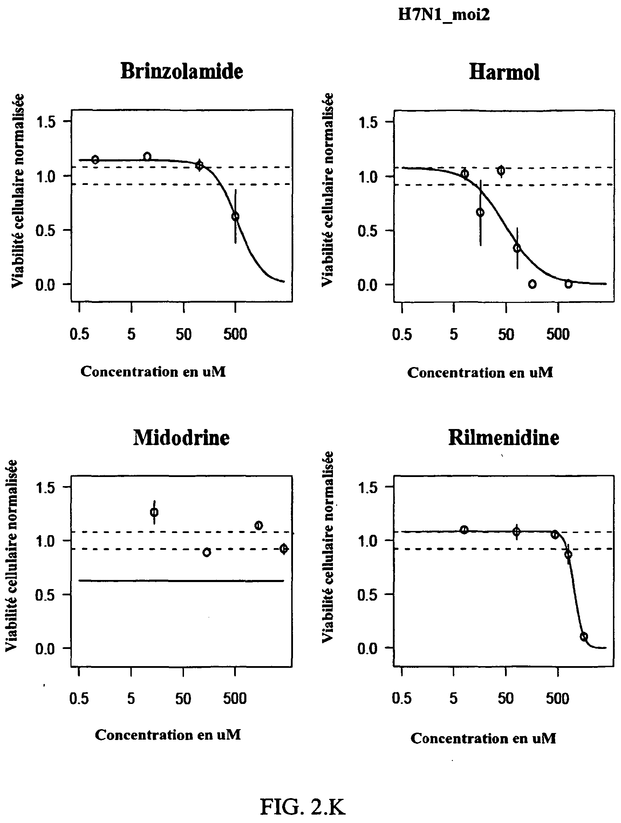

Dans chaque essai, chaque concentration de molécules est testée dans 2 puits (duplicat). La gamme de concentration a été choisie en fonction des résultats de cytotoxicité représentés dans la figure 1. Les résultats, issus de deux essais indépendants, sont représentés molécule par molécule pour chaque virus dans la figure 2.

### C. Évaluation des molécules sur l'efficacité d'infection (entrée des virus)

D'après notre hypothèse, les molécules antivirales ont un effet sur le métabolisme cellulaire en induisant un contexte cellulaire défavorable à l'infection. Pour prouver que les drogues testées agissent bien sur l'état cellulaire et non pas sur le virus et ses capacités à infecter les cellules (altération de la membrane virale, inhibiteur des glycoprotéines virales de surface HA et NA, agoniste compétiteur sur les récepteurs cellulaires des virus influenza...etc), deux essais parallèles ont été menés sur le virus H3N2.

Le premier test consiste en l'incubation pendant 15h à 37°C du virus non dilué avec différentes concentrations de molécules c tel que décrit ci-dessus. Une dilution au 1/3 dans du milieu d'infection frais est ensuite réalisée et 50 uL de cette dilution sont utilisés par puit de MW96 pour infecter des cellules A549 confluentes préalablement lavées et dans 150 uL de milieu d'infection frais. Après 15 minutes d'infection, le milieu est enlevé, les cellules sont rincées 2 fois et 200 uL de milieu est ajouté par puit. Pendant ces 15 minutes d'infection, les drogues sont à une concentration finale de c/48, on estime que cette concentration ainsi que le temps de contact sur les cellules est trop faible pour induire un effet cellulaire. Le virus est lui dilué au 1/16ème final, ce qui conduit, pour les témoins sans drogue, à un signal quantifiable et non saturant à la fin de l'essai. Au bout de 5 heures (ce qui représente un cycle d'infection), le nombre de cellules infectées est estimé en quantifiant la neuraminidase cellulaire. Si le nombre de cellules infectées est inférieur au témoin sans drogue, cela indique que le virus a été perturbé ou détruit pendant les 12h d'incubation avec la molécule.

Le deuxième test consiste en l'incubation des cellules avec les drogues pendant 15h à 37°C réalisé de la même manière que les 6 premières heures d'incubation décrites ci-dessus. Après 24h, les cellules sont lavées 2 fois dans du milieu et incubées avec 150 uL de milieu d'infection frais par puit. L'infection est réalisée pendant 15 minutes en ajoutant 50 uL/puit de virus dilué au 1/16 (donc au 1/64 final) puis les cellules sont rincées 2 fois et 200 uL de milieu est ajouté par puit. Au bout de 5 heures, le nombre de cellules infectées est estimé en quantifiant la neuraminidase cellulaire. Dans cet essai, les virus ne sont jamais en contact avec les drogues. Si les cellules préalablement traités par les molécules sont moins infectées que les témoins, cela montre que les molécules ont eu une action sur la cellule pour induire un environnement antiviral.

Pour quantifier la neuraminidase cellulaire dans ces 2 essais au bout des 5 heures d'infection, les cellules sont lavées dans du PBS puis lysées avec 25 uL/puit de Triton 1X dans du PBS pendant 1h à température ambiante sous agitation. Ces 25 uL de lysat sont incubés pendant 1h à 37°C avec 75 uL de substrats MUN (à 5 mM) dilué extemporanément au 1/75 dans du tampon réactionnel MES CaCl2. La réaction est stoppée par 150 uL de tampon glycine 50 mM et révélée par lecture au fluorimètre (λexc 355 nm et λem 460 nm).

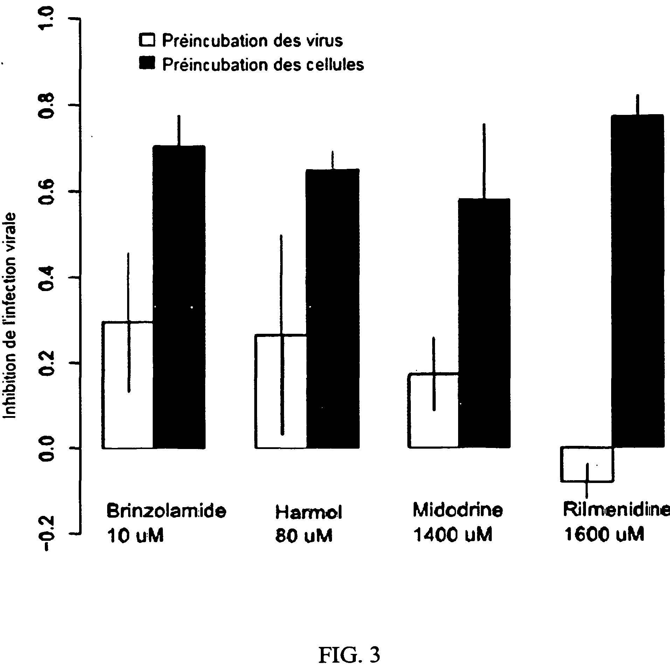

Les résultats de ces 2 tests sont présentés dans la figure 3 et montrent que l'effet des molécules décrit en B (figures 1 et 2) est toujours prédominant sur les cellules, comparé à l'effet direct sur les virus.

### D. Évaluation des molécules sur la production de virus H1N1 pandémique

Les molécules ont été évaluées pour leur activité anti-virale sur le virus H1N1 pandémique en suivant les même procédures et conditions expérimentales que décrites précédemment (A et B).

Pour ces tests, le virus a été testé à 2 moi dans 2 manipulations indépendantes. Les résultats sont représentés dans la figure 2.

Les résultats obtenus avec le virus H1N1 pandémique sont tout à fait cohérents avec ceux obtenus avec les virus choisis préalablement pour l'étude. Les molécules Harmol (molécule de référence) et Midodrine sont également caractérisées par une activité antivirale. En ce qui concerne les molécules Brinzolamide (molécule de référence) et Rilmenidine (molécule de référence), qui ne présentaient une activité anti-virale que pour certains virus, celles-ci ne semblent pas avoir d'impact sur le virus H1N1 pandémique dans les conditions testées.

### E. Données récapitulatives des propriétés de chaque molécule sur les virus

L'indice de sélectivité des molécules est le rapport entre la CC50 et l'EC50. Il est admis qu'un inhibiteur faible a un SI compris entre 2 et 10, un inhibiteur modéré a un SI entre 10 et 50 et un inhibiteur fort a un SI supérieur à 50.

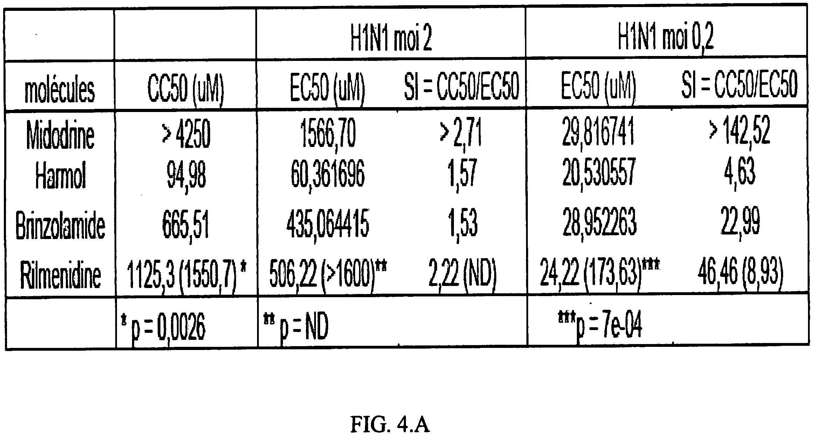

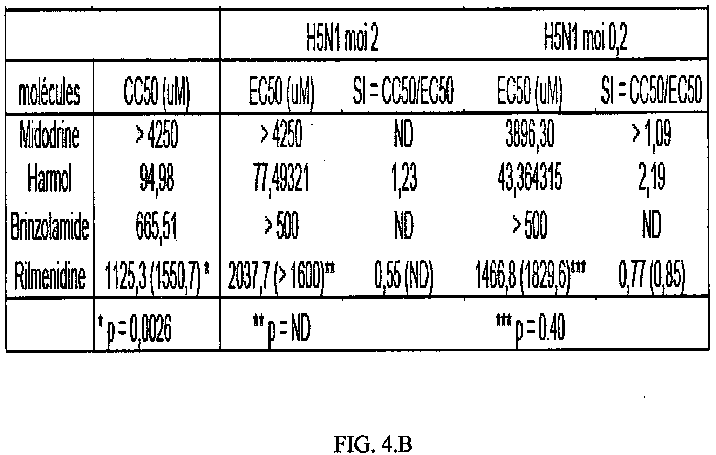

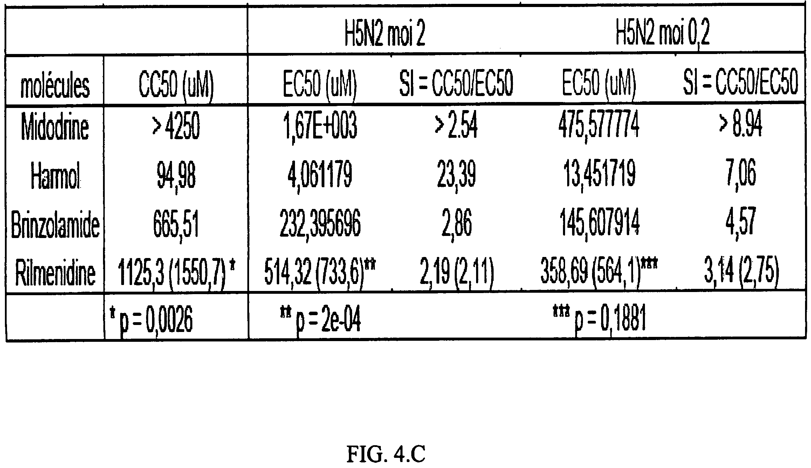

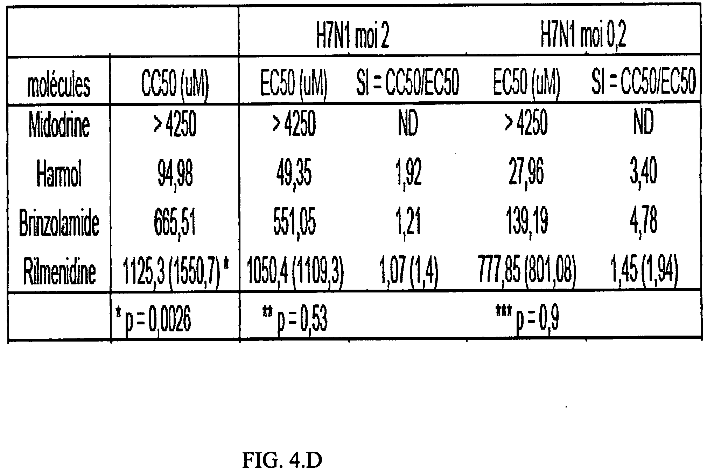

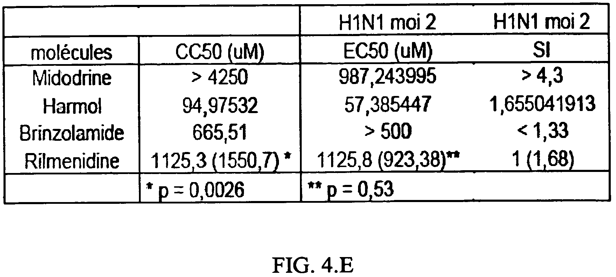

D'après nos résultats (voir figure 4), le midodrine est un inhibiteur modéré, les autres molécules sont des inhibiteurs faibles à modérés.

Le midodrine inhibe le cycle infectieux de tous les virus testés exceptés le virus H7N1. L'harmol (molécule de référence) inhide le cycle infectieux des virus H3N2, H5N2 et H5N1 et du virus H1N1 pandémique à une faible MOI.

Le brinzolamide (molécule de référence) inhibe essentiellement le cycle infectieux du virus H3N2 et des virus H1N1 et H7N1 à faibles MOI.

La rilmenidine (molécule de référence) inhibe le cycle infectieux des virus H3N2, H1N1 et H5N2.

### REFERENCES

WO 2009/047298
WO 2007/044752
WO 2009/065116

## Revendications

1. Composition pharmaceutique ou vétérinaire pour utilisation pour la prévention ou le traitement des infections virales **caractérisée en ce qu'**elle comprend au moins un composé choisi parmi la midodrine et la desglymidodrine.

2. Composition pharmaceutique ou vétérinaire selon la revendication 1 pour utilisation pour la prévention ou le traitement des infections par les virus de la grippe.

3. Composition pharmaceutique ou vétérinaire pour utilisation pour la prévention ou le traitement des infections virales selon l'une des revendications précédentes, **caractérisée en ce qu'**elle comprend en outre un véhicule pharmaceutique approprié.

4. Composition pharmaceutique ou vétérinaire pour utilisation pour la prévention ou le traitement des infections virales selon l'une des revendications précédentes, **caractérisée en ce qu'**elle comprend en outre un autre agent anti-viral ou anti-grippal.

5. Composition pharmaceutique ou vétérinaire pour utilisation pour le traitement des infections virales selon la revendication 4, **caractérisée en ce que** l'agent anti-viral ou anti-grippal est choisi parmi l'oseltamivir, le zanamivir, le peramivir, l'amantadine, la rimantadine, la ribavirine et l'arbidol.

6. Produit comprenant au moins un composé choisi parmi la midodrine et la desglymidodrine, ainsi qu'un autre agent antiviral comme produit de combinaison pour une utilisation simultanée, séparée ou étalée dans le temps dans la prévention et le traitement des infections virales.

## Patentansprüche

1. Pharmazeutische oder veterinärmedizinische Zusammensetzung für die Verwendung zur Prävention oder Behandlung von Virusinfektionen, **dadurch gekennzeichnet, dass** sie wenigstens eine Verbindung umfasst, die ausgewählt ist aus Midodrin und Desglymidodrin.

2. Pharmazeutische oder veterinärmedizinische Zusammensetzung gemäß Anspruch 1 für die Verwendung zur Prävention oder Behandlung von Infektionen durch Grippeviren.

3. Pharmazeutische oder veterinärmedizinische Zusammensetzung für die Prävention oder Behandlung von Virusinfektionen gemäß einem der voranstehenden Ansprüche, **dadurch gekennzeichnet, dass** sie darüber hinaus einen geeigneten pharmazeutischen Träger umfasst.

4. Pharmazeutische oder veterinärmedizinische Zusammensetzung für die Prävention oder Behandlung von Virusinfektionen gemäß einem der voranstehenden Ansprüche, **dadurch gekennzeichnet, dass** sie darüber hinaus einen weiteren antiviralen oder antigrippalen Wirkstoff umfasst.

5. Pharmazeutische oder veterinärmedizinische Zusammensetzung zur Behandlung von Virusinfektionen gemäß Anspruch 4, **dadurch gekennzeichnet, dass** der antivirale oder antigrippale Wirkstoff ausgewählt ist aus Oseltamivir, Zanamivir, Peramivir, Amantadin, Rimantadin, Ribavirin und Arbidol.

6. Produkt, umfassend wenigstens eine Verbindung, die ausgewählt ist aus Midodrin und Desglymidodrin sowie einem weiteren antiviralen Wirkstoff als Kombinationsprodukt für eine gleichzeitige, separate oder zeitlich aufgeteilte Verwendung bei der Prävention und der Behandlung von Virusinfektionen.

## Claims

1. A pharmaceutical or veterinary composition for use to prevent or treat viral infections **characterized in that** it comprises at least one compound selected from midodrine and desglymidodrine.

2. The pharmaceutical or veterinary composition according to claim 1 for use to prevent or treat influenza virus infections.

3. The pharmaceutical or veterinary composition for use to prevent or treat viral infections according to one of the preceding claims, **characterized in that** it further comprises a suitable pharmaceutical carrier.

4. The pharmaceutical or veterinary composition for use to prevent or treat viral infections according to one of the preceding claims, **characterized in that** it further comprises another antiviral or anti-influenza agent.

5. The pharmaceutical or veterinary composition for use to treat viral infections according to claim 4, **characterized in that** the antiviral or anti-influenza agent is selected from oseltamivir, zanamivir, peramivir, amantadine, rimantadine, ribavirin and arbidol.

6. A product comprising at least one compound selected from midodrine and desglymidodrine as well as another antiviral agent as a combination product for simultaneous, separated or staggered use in the prevention and treatment of viral infections.
